(19) Europäisches Patentamt / European Patent Office / Office européen des brevets

(11) **EP 3 795 985 B1**

(12) **EUROPEAN PATENT SPECIFICATION**

(45) Date of publication and mention of the grant of the patent:
**04.09.2024 Bulletin 2024/36**

(21) Application number: **19804488.5**

(22) Date of filing: **26.02.2019**

(51) International Patent Classification (IPC):
**G01N 23/2055** (2018.01)   **C01B 32/182** (2017.01)
**C01B 32/19** (2017.01)   **C01B 32/20** (2017.01)
**G01N 33/24** (2006.01)

(52) Cooperative Patent Classification (CPC):
**G01N 23/2055; C01B 32/20; G01N 33/24;**
G01N 2223/0566; G01N 2223/345; G01N 2223/605

(86) International application number:
**PCT/JP2019/007216**

(87) International publication number:
**WO 2019/220737 (21.11.2019 Gazette 2019/47)**

(54) **DISTINGUISHING METHOD OF GRAPHENE PRECURSOR, AND DISTINGUISHING APPARATUS AND DISTINGUISHING PROGRAM THEREOF**

UNTERSCHEIDUNGSVERFAHREN, -VORRICHTUNG UND -PROGRAMM FÜR GRAPHEN-VORLÄUFERMATERIALIEN

PROCÉDÉ, DISPOSITIF ET PROGRAMME DE DISTINCTION DES MATERIAUX PRÉCURSEUR DE GRAPHÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.05.2018 JP 2018093324**

(43) Date of publication of application:
**24.03.2021 Bulletin 2021/12**

(73) Proprietor: **Rigaku Corporation
Akishima-shi
Tokyo 196-8666 (JP)**

(72) Inventors:
• **SASAKI, Akito
Akishima-shi, Tokyo 196-8666 (JP)**
• **MUROYAMA, Norihiro
Akishima-shi, Tokyo 196-8666 (JP)**

(74) Representative: **Lambacher, Michael et al
V. Füner Ebbinghaus Finck Hano
Patentanwälte
Mariahilfplatz 3
81541 München (DE)**

(56) References cited:
**WO-A1-2016/002254   JP-A- H11 322 433
JP-B1- 5 697 067**

• **MOHINDAR S. SEEHRA ET AL: "Detection and quantification of 2H and 3R phases in commercial graphene-based materials", CARBON, vol. 95, 4 September 2015 (2015-09-04), GB, pages 1 - 16, XP055654386, ISSN: 0008-6223, DOI: 10.1016/j.carbon.2015.08.109**
• **ANONYMOUS: "Integrated intensity - GISAXS", 20 January 2015 (2015-01-20), pages 1 - 7, XP055887077, Retrieved from the Internet <URL:http://gisaxs.com/index.php/Integrated_intensity> [retrieved on 20220203]**
• **MOHINDAR S SEEHRA; USHA K GEDDAM; DIANE SCHWEGLER-BERRY; ALEKSANDR B STEFANIAK: "Detection and quantification of 2H and 3R phases in commercial graphene-based materials", CARBON, vol. 95, 4 September 2015 (2015-09-04), pages 1 - 16, XP055654386, ISSN: 0008-6223, DOI: 10.1016/j.carbon.2015.08.109**

EP 3 795 985 B1

- KATSUAKI SUGAWARA; NORIFUMI YAMAMURA; KEITA MATSUDA; WATARU NORIMATSU; MICHIKO KUSUNOKI; TAKAFUMI SATO; TAKASHI TAKAHASHI: "Selective fabrication of free-standing ABA and ABC trilayer graphen with/without Dirac-cone energy bands", NPG ASIA MATERIALS, vol. 10, no. 2, e466, 9 February 2018 (2018-02-09), pages 1 - 5, XP055654392, ISSN: 1884-4049, DOI: 10.1038/am.2017.238
- ZHU-JUN WANG; JICHEN DONG; YI CUI; GYULA ERES; OLAF TIMPE; QIANG FU; FENG DING; SCHLOEGL R; MARC-GEORG WILLINGER: "Stacking sequence and inter layer coupling in few-layer graphene revealed by in situ imaging", NATURE COMMUNICATIONS, vol. 7, 13256, 19 October 2016 (2016-10-19), pages 1 - 12, XP055654393, DOI: 10.1038/ncomms13256
- CHUN HUNG LUI; ZHIQIANG LI; ZHEYUAN CHEN; PAUL V KLIMOV; LOUIS E BRUS; TONY F HEINZ: "Imaging Stacking Order in Few-Layer Graphene", NANO LETTERS, vol. 11, no. 1, 1 December 2010 (2010-12-01), pages 164 - 169, XP055654394, ISSN: 1530-6984, DOI: 10.1021/nl1032827
- SEEHRA MOHINDAR S; NARANG VISHAL; GEDDAM USHA K; STEFANIAK ALEKSANDR B: "Correlation between X-ray diffraction and Raman spectra of 16 commercial graphene-based materials and their resulting classification", CARBON, vol. 111, 8 October 2016 (2016-10-08), pages 380 - 385, XP029808435, ISSN: 0008-6223, DOI: 10.1016/j.carbon.2016.10.010
- FUMIHIKO MATSUI; RYO ISHII; HIROYUKI MATSUDA; MAKOTO MORITA; SATOSHI KITAGAWA; TOMOHIRO MATSUSHITA; SHINJI KOH; HIROSHI DAIMON: "Characterizing Edge and Stacking Structures of Exfoliated Graphene by Photoelectron Diffraction", JAPANESE JOURNAL OF APPLIED PHYSICS, vol. 52, 110110, 28 October 2013 (2013-10-28), pages 1 - 5, XP055654516, ISSN: 0021-4922, DOI: 10.7567/JJAP.52.110110
- ZHANG, JI-LIANG; SHAN, GUANG-CUN: "Stacking control in graphene-based materials: a promising way for fascinating physical properties", ARXIV.ORG, 7 February 2019 (2019-02-07), pages 1 - 4, XP081026538, DOI: 10.1007/s11467-018-0871-2
- "Graphite/graphene analysis index", RIGAKU CORPORATION'S NEW ARRIVAL INFORMATION, 3 October 2018 (2018-10-03), XP055654531, Retrieved from the Internet <URL:https://www.rigaku.com/ja/newsline>
- ANONYMOUS: "Graphite/graphene analysis index calculation software GG Index", RIGAKU JOURNAL, vol. 50, no. 1, 21 April 2019 (2019-04-21), pages 39 - 41, XP009523909, ISSN: 1883-8456
- ANONYMOUS: "Graphite/graphene analytical index calculator GG Index", RIGAKU JOURNAL, vol. 35, no. 1, 4 March 2019 (2019-03-04), pages 38 - 40, XP009523912, ISSN: 2187-9974

## Description

### TECHNICAL FIELD

The present invention relates to a distinguishing method of a graphene precursor with respect to graphite raw material in which a hexagonal crystal system graphite layer and a rhombohedral crystal system graphite layer are mixed, and to a distinguishing apparatus and a distinguishing program thereof.

### BACKGROUND ART

Graphene is a new carbon material that was discovered in 2004 by both Dr. Geim and Dr. Novosolv at University of Manchester in England. It was found out by the two of them that graphene is a material with extremely superior physical properties compared to a conventional material, graphene exhibited a 100 times or larger strength than steel; exhibited a heat conducting characteristic and an electrical property of 10 to 100 times metal; and further a complete barrier property of not passing through various materials such as gas, liquid, etc. since a plane crystal structure is very small; and so forth.

Through this great discovery, Nobel Prize in Physics was awarded jointly to the two of them in 2010, just 6 years after the discovery in breaking of the custom of Nobel Prize. Then, the research toward the next generation on subjects such as graphene-based semiconductors, communication elements, electronic devices, batteries and so forth has been actively progressing, and in Europe and America, the 21st century has become so far as to be called "the age of Graphene Valley" in parody of Silicon Valley in California, USA, that has been developed from the late 20th century with high performance of silicon semiconductors.

Graphene is an ultrathin nano-carbon material, similar in thinness to a single atom thick. A specific crystal structure in which carbon atoms are bonded in a honeycomb shape to form a two-dimensional plane is provided, and thus the graphene exhibits very high electron mobility, and also exhibits excellent physical properties such as an optical property, a thermal property, a dynamic property and so forth. Accordingly, the research and development of graphene has been actively progressing as a material of the next generation. There are several methods of synthesizing graphene, one of which is a method of acquiring graphene from graphite that is also a material of pencil. Graphite is a material having three-dimensional structure where two-dimensional graphene is attached thereon in multiple layers, and thus the graphene can be obtained by peeling this off (Refer to Patent Document 1).

An advantage of the method of peeling graphene off from graphite is that production cost can be largely reduced. In comparison to CVD (chemical vapor deposition method) as a major graphene preparation method other than the peeling method, the productivity of the peeling method is obviously high. CVD is a method in which a substrate such as copper or the like is placed inside a vacuum chamber, and gas containing carbon as a material of graphene flows thereto. Graphene of a large area with high quality can be film-formed on a substrate, however cost becomes high due to use of a vacuum environment, and thus it costs tens of dollars to hundreds of dollars to prepare graphene of 1 m$^2$, though depending on its quality. On the other hand, according to the peeling method, at the same cost of tens of dollars to hundreds of dollars, graphene of a weight of approximately 1 kg can be obtained. Since graphene is ultrathin and ultralight, and its surface area per unit weight is a huge value of approximately 2500 m$^2$/g, a weight of 1 kg thereof is equivalent to CVD graphene with an area of 2,500,000 m$^2$, film-formed on a substrate. That is, if a comparison is made in weight, there is a significant, 2.5 million-fold difference between CVD and the peeling method in terms of cost.

On the other hand, the crystal structure of graphite can be figured out by a method (X-ray diffraction) of examining a diffraction phenomenon generated by scattering or interference of X-rays when a sample is irradiated with the X-rays. An existence ratio of each crystal structure can be distinguished from integrated intensity ratios of respective peaks by a powder X-ray diffraction method performing peak resolution on the acquired profiles (Refer to Non-Patent Document 1). From patent Document 2 a technique for distinguishing a graphene precursor from a graphite raw material is known. The technique is based on the evaluation of XRD patterns. More specifically, the intensities of the (101)-diffraction peaks associated with the hexagonal system graphite layer and the rhombohedral system graphite layer are evaluated to determine whether a graphite raw material is a suitable graphene precursor or not.

### PRIOR ART DOCUMENT

### PATENT DOCUMENT

Patent Document 1: Re-publication of PCT International Publication No. 2016-038692
Patent Document 2: JP 5 697067 B1

**NON-PATENT DOCUMENT**

Non-Patent Document 1: R. A. Young, "The Rietveld Method", International Union of Crystallography Oxford University Press 1995, p. 255 - 275

**SUMMARY OF THE INVENTION**

**PROBLEMS TO BE SOLVED BY THE INVENTION**

As to graphene produced by CVD, it is estimated that small-quantity production for high-value-added applications is carried out from now on. However, other than such applications, it appears that it is certain that graphene is mass-produced for various industrial applications by the peeling method.

In this case, regarding profiles via the powder X-ray diffraction method, obtained from graphite raw material, it becomes important to evaluate suitability as a graphene precursor by distinguishing an existence ratio of each crystal structure from integrated intensities of two peaks in the vicinity of a diffraction angle $2\theta$ of 42 to 46°.

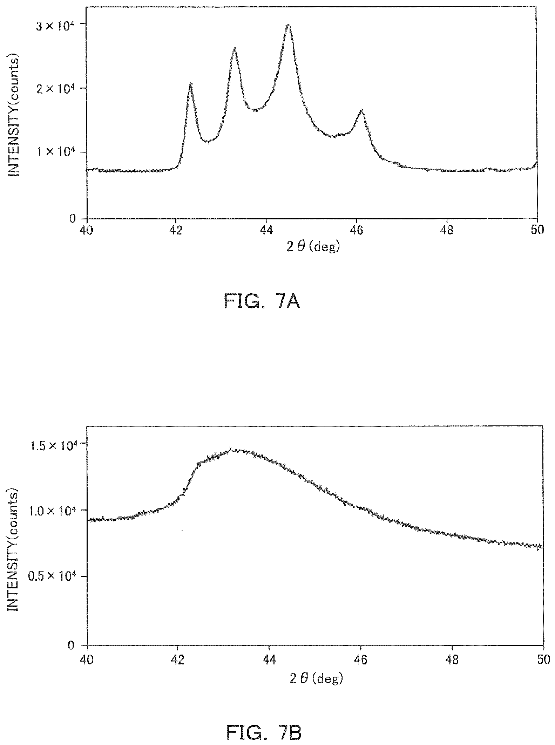

FIGS. 7A and 7B are diagrams showing profiles of powder X-ray diffraction patterns, whose peaks are distinguishable and undistinguishable from the appearance thereof respectively. As shown in FIG. 7A, when the peak can be clearly distinguished from the appearance of the profile, the peak separation is able to be performed sufficiently by peak profile fitting.

However, according to, for example, the powder X-ray diffraction pattern with respect to graphite raw material derived from natural graphite, as shown in FIG. 7B, a gently-sloping mountain in which the profile appearance includes the entire angle range is shown, and thus there is a case where each peak is not distinguishable from the appearance. In such a case, no peak separation is enabled, even though simply applying the peak profile fitting thereto.

The present invention has been made in view of such a situation, and it is an object to provide a distinguishing method of a graphene precursor, that surely enables peak separation by peak profile fitting with a powder X-ray diffraction pattern with respect to graphite raw material, and also provide a distinguishing apparatus and a distinguishing program thereof.

**MEANS TO SOLVE THE PROBLEMS**

(1) In order to achieve the above-described object, it is a feature that the distinguishing method of a graphene precursor according to the present invention is a distinguishing method of a graphene precursor with respect to graphite raw material in which a hexagonal crystal system graphite layer and a rhombohedral crystal system graphite layer are mixed, the method comprising the steps of specifying a zero-point of a diffraction angle from at least one peak position of a diffracted beam by lattice planes of c-axis direction for powder X-ray diffraction data of the graphite raw material; calculating peak positions specific to the hexagonal crystal system graphite layer and the rhombohedral crystal system graphite layer from the specified zero-point, by assuming that an interplanar spacing between graphene sheets forming the graphite raw material is the same in any graphite layer mixed in the graphite raw material and that a crystal structure inside the graphene sheets is the same in any graphite layer mixed in the graphite raw material, wherein the a-axis and b-axis lengths of the hexagonal crystal system graphite layer 2H in the graphite raw material are equal to the a-axis and b-axis lengths of the rhombohedral crystal system graphite layer 3RH therein, respectively; and the c-axis length of the hexagonal crystal system graphite layer 2H is 2/3 times the c-axis length of the rhombohedral crystal system graphite layer 3RH; calculating integrated intensities at the calculated peak positions; determining whether or not a rate of an integrated intensity value of a 101 diffracted beam of the hexagonal crystal system graphite layer to an integrated intensity value of a 101 diffracted beam of the rhombohedral crystal system graphite layer at the calculated peak positions is the same as or higher than a predetermined value, based on the calculated integrated intensities; and determinably outputting that the graphite raw material is the graphene precursor when it is determined that the rate with regard to the integrated intensity values is the same as or higher than the predetermined value, and that the graphite raw material is not the graphene precursor when it is determined that the rate with regard to the integrated intensity values is lower than the predetermined value.

In this manner, the peak separation can be surely enabled by a powder X-ray diffraction pattern with respect to graphite raw material. As a result of this, a ratio of the hexagonal crystal system graphite layer and the rhombohedral crystal system graphite layer in the graphite raw material can be measured accurately, and thus it can be accurately and precisely distinguished whether or the graphite raw material is a graphene precursor or not. Then, it becomes possible to produce graphene at low cost by using the peeling method.

(2) Further, it is a feature that the distinguishing method of a graphene precursor according to the present invention is the method, wherein in the step of specifying the zero-point of the diffraction angle, a peak position of a diffracted beam with the graphene sheets as lattice planes is used, by assuming that the interplanar spacing between a plurality of the graphene sheet that forms the graphite raw material is the same in any structure mixed in the graphite raw material. In this manner, the zero-point of the diffraction angle can be specified from the peak position.

(3) Further, it is a feature that the distinguishing method of a graphene precursor according to the present invention is the method, wherein it is distinguished whether or not P3/(P3 + P4) is 0.31 or more, when the calculated integrated intensity value of the 101 diffracted beam of the rhombohedral crystal system graphite layer represents P3, and the calculated integrated intensity value of the 101 diffracted beam of the hexagonal crystal system graphite layer represents P4. In this manner, the graphite raw material for which a graphene peeling method is applicable can be clearly distinguished by using specific numerical values.

(4) Further, it is a feature that the distinguishing apparatus according to the present invention is a distinguishing apparatus of a graphene precursor with respect to graphite raw material in which a hexagonal crystal system graphite layer and a rhombohedral crystal system graphite layer are mixed, the apparatus comprising a zero-point specification section that specifies a zero-point of a diffraction angle from at least one position of a diffracted beam by lattice planes of c-axis direction for powder X-ray diffraction data of the graphite raw material; a peak position calculation section that calculates peak positions specific to the hexagonal crystal system graphite layer and the rhombohedral crystal system graphite layer from the specified zero-point, by assuming that an interplanar spacing between graphene sheets that form the graphite raw material is the same in any graphite layer mixed in the graphite raw material and that a crystal structure inside the graphene sheets is the same in any graphite layer mixed in the graphite raw material, wherein the a-axis and b-axis lengths of the hexagonal crystal system graphite layer 2H in the graphite raw material are equal to the a-axis and b-axis lengths of the rhombohedral crystal system graphite layer 3RH therein, respectively; and the c-axis length of the hexagonal crystal system graphite layer 2H is 2/3 times the c-axis length of the rhombohedral crystal system graphite layer 3RH; an integrated intensity calculation section that calculates integrated intensities at the calculated peak positions; a determination section (270) that determines whether or not a rate of an integrated intensity value of a 101 diffracted beam of the hexagonal crystal system graphite layer to an integrated intensity value of a 101 diffracted beam of the rhombohedral crystal system graphite layer at the calculated peak positions is the same as or higher than a predetermined value, based on the calculated integrated intensities; and an output section (280) that determinably outputs that the graphite raw material is the graphene precursor when it is determined that the rate with regard to the integrated intensity values is the same as or higher than the predetermined value, and that the graphite raw material is not the graphene precursor when it is determined that the rate with regard to the integrated intensity values is lower than the predetermined value. In this manner, the peak separation is surely enabled by a powder X-ray diffraction pattern with respect to graphite raw material, and thus it can be distinguished whether the material is a graphene precursor or not.

(5) Further, it is a feature that the distinguishing program according to the present invention is a program of distinguishing a graphene precursor with respect to graphite raw material in which a hexagonal crystal system graphite layer and a rhombohedral crystal system graphite layer are mixed, the program causing a computer to execute the processes of specifying a zero-point of a diffraction angle from at least one position of a diffracted beam by lattice planes of c-axis direction for powder X-ray diffraction data of the graphite raw material; calculating peak positions specific to the hexagonal crystal system graphite layer and the rhombohedral crystal system graphite layer from the specified zero-point, by assuming that an interplanar spacing between graphene sheets that form the graphite raw material is the same in any graphite layer mixed in the graphite raw material and that a crystal structure inside the graphene sheets is the same in any graphite layer mixed in the graphite raw material, wherein the a-axis and b-axis lengths of the hexagonal crystal system graphite layer 2H in the graphite raw material are equal to the a-axis and b-axis lengths of the rhombohedral crystal system graphite layer 3RH therein, respectively; and the c-axis length of the hexagonal crystal system graphite layer 2H is 2/3 times the c-axis length of the rhombohedral crystal system graphite layer 3RH; calculating integrated intensities at the calculated peak positions; determining whether or not a rate of an integrated intensity value of a 101 diffracted beam of the hexagonal crystal system graphite layer to an integrated intensity value of a 101 diffracted beam of the rhombohedral crystal system graphite layer at the calculated peak positions is the same as or higher than a predetermined value, based on the calculated integrated intensities; and determinably outputting that the graphite raw material is the graphene precursor when it is determined that the rate with regard to the integrated intensity values is the same as or higher than the predetermined value, and that the graphite raw material is not the graphene precursor when it is determined that the rate with regard to the integrated intensity values is lower than the predetermined value.

## EFFECT OF THE INVENTION

According to the present invention, the peak separation can be surely enabled using peak profile fitting by a powder X-ray diffraction pattern with respect to graphite raw material.

## BRIEF DESCRIPTION OF THE DRAWINGS

FIGS. 1A and 1B are diagrams showing crystal structures of a hexagonal crystal system graphite layer and a rhombohedral crystal system graphite layer in graphite, respectively.

FIG. 2 is a schematic diagram showing a configuration of a system of distinguishing a graphene precursor according to the present invention.

FIG. 3 is a block diagram showing a functional configuration of a distinguishing apparatus of a graphene precursor according to the present invention.

FIG. 4 is a flowchart showing a distinguishing method of a graphene precursor according to the present invention.

FIGS. 5A and 5B are graphs schematically showing profiles having a powder X-ray diffraction pattern of graphite raw material with respect to 2θ of 0 to 90° and 2θ of 40 to 45°, respectively.

FIG. 6 is a diagram showing a table that shows experimental results of each sample.

FIGS. 7A and 7B are diagrams showing profiles of powder X-ray diffraction patterns, whose peaks are distinguishable and undistinguishable from the appearance thereof, respectively.

## DETAILED DESCRIPTION OF EMBODIMENTS

Next, embodiments of the present invention will be described referring to the drawings. In order to facilitate understanding of the description, reference number indicating the same constituent element is used as same and overlapping descriptions will be omitted in each drawing.

[Graphene precursor]

The hexagonal crystal system graphite layer abundantly contained in natural graphite is very stable, and thus energy exceeding Van der Waals force between graphene layers is given to peel off graphene thereof. Then, the energy required for peeling off is inversely proportional to the cube of the thickness. Accordingly, graphene is peeled off by weak physical force such as ultrasonic wave or the like because of being very weak in the state of being thick where layers are countlessly stacked, however, very large energy is required when being peeled off from thin graphite in some degree.

According to the peeling method by which graphene is peeled off from graphite to be produced, high efficiency can be further achieved by using "graphene precursor" that is suitable therefor. The graphene precursor is graphite having a specific crystal structure, and in contrast, when performing a conventional peeling process (such as ultrasonic wave, supercriticality, high-pressure spraying, collision, grinding, oxidation, reduction, microwave (in ionic liquid), kneading and so forth), the productivity is improved by 2.5 million times in theoretical value, as an estimate, compared to producing graphene by a CVD method.

The graphene precursor having lower crystallinity than that of conventional graphite is easy to be peeled off, and thus a large amount of high-concentration graphene can be produced without a concentration process required in the conventional peeling method. This technique enables mass production of graphene (an annual production capacity of 100 to 10,000 tons or more), and supplying sufficient materials also in fields of secondary battery, capacitor, resin, rubber composite, composite building materials and so forth for which relatively inexpensive nanomaterials (20 to 100 dollars per kg) is required. It appears that the material development utilizing graphene will be further accelerated from now on, and thus if the above-described method is standardized, it becomes further important to distinguish a graphene precursor for companies and institutions that supply the graphene precursor that is raw material thereof.

[Principle of distinguishing method]

That kind of graphene precursor is configured to contain a rhombohedral crystal system graphite layer 3RH at a fixed ratio or more to a hexagonal crystal system graphite layer 2H. In addition, the hexagonal crystal system can be anything comprising crystal structure of an almost hexagonal crystal, which may be a perfect hexagonal crystal; a hexagonal crystal whose crystal structure is partially deformed; or a hexagonal crystal whose part or all is distorted. The same applies to rhombohedral crystal system.

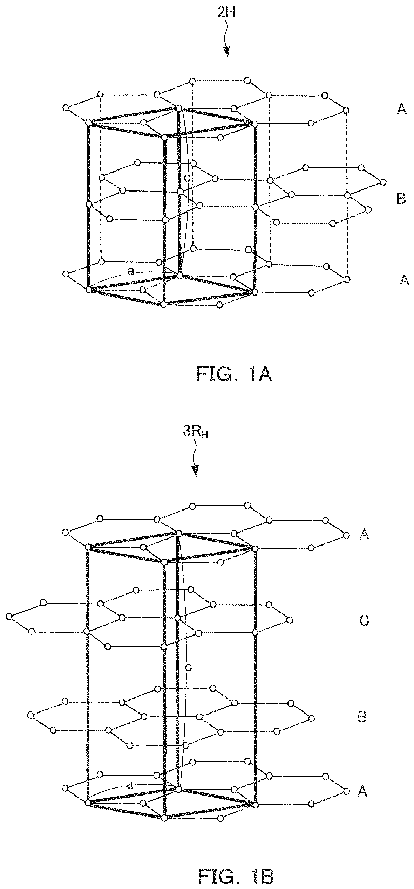

FIGS. 1A and 1B are diagrams showing crystal structures of a hexagonal crystal system graphite layer 2H and a rhombohedral crystal system graphite layer 3RH in graphite, respectively. It is known that natural graphite is classified into three kinds of crystal structures: a hexagonal crystal system graphite layer, a rhombohedral crystal system graphite layer and disorder, by how layers are stacked. As shown in FIG. 1A, regarding the hexagonal crystal system graphite layer 2H, the layers are stacked in order of ABABAB · · to form a crystal structure, and as shown in FIG. 1B, regarding the rhombohedral crystal system graphite layer 3RH, the layers are stacked in order of ABCABCABC ·· to form a crystal structure. Provided that the rhombohedral crystal system graphite layer 3RH is actually classified into a rhombohedral crystal, however as shown in FIG. 1B, it is regarded as forming a hexagonal crystal in a pseudo manner, thereby being represented by using an axis of a hexagonal crystal system (that is, any crystal system below is represented by using an a-axis, a b-axis and a c-axis.)

The natural graphite has almost no rhombohedral crystal system graphite layer at the mining stage, however a ratio thereof is increased by being pulverized and the like at the purification stage, and thus the rhombohedral crystal system graphite layer is present in a natural graphite-based carbon material by around 14%. The ratio of the rhombohedral crystal system graphite layer can be increased by processing the natural graphite by physical force or heat, thereby further enhancing the ratio by using radio-wave force together.

The crystal structure of graphite can be figured out by a method (X-ray diffraction) of examining a diffraction phenomenon caused by scattering/interference of X-ray when X-ray is irradiated to a sample thereof. Accordingly, it can be determined whether the sample is a graphene precursor or not via checking whether the ratio of the rhombohedral crystal system graphite layer is same as or higher than a predetermined value, by comparing integrated intensities of peaks specific to respective crystal structures measured using X-ray diffraction. For example, this can be determined by the following numerical formula (1).

$$31\% \leq \text{Rate (3RH)} = P3/(P3 + P4) \quad \cdots \quad (1)$$

P3: An integrated intensity value ($2\theta = 43$ or a level thereof) of the rhombohedral crystal system graphite layer (101)
P4: An integrated intensity value ($2\theta = 44$ or a level thereof) of the hexagonal crystal system graphite layer (101)

The integrated intensity of a peak in a powder X-ray diffraction pattern can be calculated by giving initial values (position, width, height and so forth) of the peak; setting a restricting condition so as to prevent divergence of parameters during being refined; and performing profile fitting. At this time, since a converging position of the parameters varies in accordance with setting of the initial values, it is required for calculating a precise integrated intensity to utilize proper initial values.

Then, in the present invention, given is an assumption that "graphite raw material contains two kinds of polymorphisms: a hexagonal crystal system graphite layer 2H and a rhombohedral crystal system graphite layer 3RH, however any of them is one obtained in a closed synthetic environment (production environment), and thus an interplanar spacing between graphene sheets is the same and a structure inside the graphene sheets is the same in any structure".

From those described above, the following relationships (1) and (2) are satisfied for lattice constants in two crystal structures.

(1) The a-axis and b-axis lengths of 2H are equal to the a-axis and b-axis lengths of 3RH, respectively.
(2) The c-axis length of 2H is 2/3 times the c-axis length of 3RH.

The initial value at the peak position is determined so as to satisfy these two relationships above. Since the peak position is finally refined, there is no problem even if a value slightly shifted from a precise value is used as an initial value. In addition, the c-axis length may be obtained by calculation based on the peak position of a 002 plane appearing at approximately 26°.

[Configuration of system of distinguishing graphene precursor]

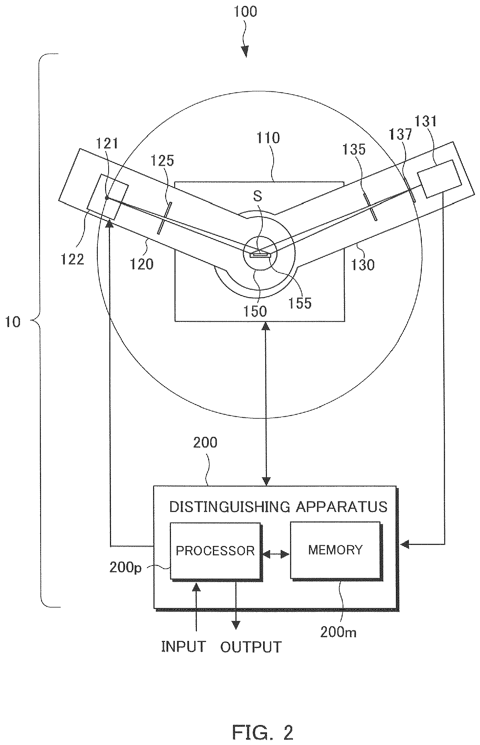

Next, the system capable of distinguishing a graphene precursor is explained, based on the assumption as described above. FIG. 2 is a schematic diagram showing a configuration of a distinguishing system 10 of a graphene precursor. As shown in FIG. 2, the distinguishing system 10 of a graphene precursor comprises an X-ray diffractometer 100 and a distinguishing apparatus 200. The X-ray diffractometer 100 and the distinguishing apparatus 200 are connected to each other in order to be able to transmit/receive information. The connection comprises information transmission with a storage medium, regardless of being wired and wireless.

[X-ray diffractometer]

An X-ray diffractometer 100 is a device of measuring a powder X-ray diffraction pattern by irradiating X-rays of a specific wavelength such as CuKα1 rays to a powder sample S. The X-ray diffractometer 100 is provided with a goniometer 110, and the goniometer 110 is provided with an X-ray source arm 120, a detector arm 130 and a sample stage 150. The X-ray source arm 120 supports an X-ray source 121, and the detector arm 130 supports an X-ray detector 131.

The sample stage 150 provided at the center portion of the goniometer 110 supports a sample holder 155 to be able to be attached/detached. The powder sample S is filled inside a recess formed in the sample holder 155.

Each of the X-ray source arm 120 and the detector arm 130 are independently rotatable with the same rotation axis as a center. The rotation axis is set so as to pass on the surface of the sample S supported by the goniometer 110.

An X-ray generator 122 and a divergence slit 125 are fixed on the X-ray source arm 120. Other optical elements, for example, a solar slit for parallelizing X-rays, a monochromator for taking characteristic X-rays out of X-rays including X-rays of various wavelengths, and so forth can also be arranged on the X-ray source arm 120, as needed.

A filament that generates thermal electrons via current application and a target arranged to be opposed thereto are provided inside the X-ray generator 122. A region where the thermal electrons collide with the target becomes the X-ray source 121.

A scattering slit 135, a receiving slit 137, and the X-ray detector 131 are provided on the detector arm 130. The scattering slit 135 prevents scattered X-rays from entering the X-ray detector 131. The receiving slit 137 prevents X-rays other than focused X-rays from entering the X-ray detector 131. The X-ray detector 131 is constituted of, for example, a zero-dimensional X-ray detector, and outputs a signal corresponding to the quantity of received X-rays, and an intensity detection circuit calculates the X-ray intensity based on the output signal. Either an one-dimensional X-ray detector or a two-dimensional X-ray detector may also be used as a X-ray detector. In addition, the X-ray diffractometer 100 can be any X-ray diffractometer as long as it is provided with Bragg-Brentano optics and able to measure a powder X-ray diffraction pattern, and utilized may be a configuration that is different from one described above, such as one in which the X-ray detector 131 is arranged on a Rowland circle, and so forth.

[Distinguishing apparatus of graphene precursor]

The distinguishing apparatus 200 of a graphene precursor comprising a processor 200p and a memory 200m in hardware configuration is constituted from, for example, PC. The distinguishing apparatus 200 can calculate the integrated intensity of the peak for each crystal structure concerning graphite raw material in which the hexagonal crystal system graphite layer 2H and the rhombohedral crystal system graphite layer 3RH are mixed, based on the acquired powder X-ray diffraction pattern with a program stored in the memory 200m, that is executed by processor 200p. As a result of this, it enables a user to distinguish whether it is a graphene precursor or not.

The distinguishing apparatus 200 receives input from a user and then outputs a processing result to the user. The input from the user is enabled by an operation device such as a keyboard, a mouse, a touch panel or the like, and can be displayed by an output device such as a display or the like.

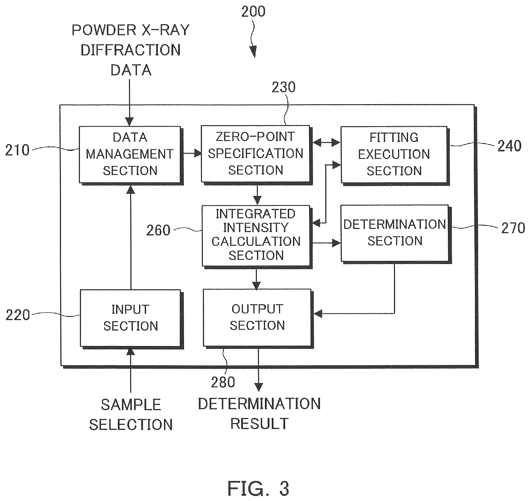

FIG. 3 is a block diagram showing a functional configuration of the distinguishing apparatus 200 of a graphene precursor. As shown in FIG. 3, the distinguishing apparatus 200 comprises a data management section 210, an input section 220, a zero-point specification section 230, an integrated intensity calculation section 260, a fitting execution section 240, a determination section 270 and an output section 280.

The data management section 210 stores and manages powder X-ray diffraction data received from the X-ray diffractometer 100 for each sample. The input section 220 receives input from an operation device such as a keyboard, a mouse, a touch panel or the like that is operated by a user. For example, when a sample is designated via the operation device by the user, the designation is received by the input section 220, and powder X-ray diffraction data of the designated sample is defined as a processing object by the data management section 210. In addition, the powder X-ray diffraction data indicates data obtained by a powder X-ray diffraction method; and the powder X-ray diffraction pattern or the profile indicates one in which the acquired data is recognized as a diffraction pattern or a profile; and although how to recognize it differs therefrom, substantial object data are identical thereto.

The zero-point specification section 230 specifies a zero-point of a diffraction angle for the powder X-ray diffraction data of graphite raw material. It is preferred to specify a zero-point of a diffraction angle from a peak position of a diffracted beam by lattice planes in a c-axis direction common to the hexagonal crystal system graphite layer 2H and the rhombohedral crystal system graphite layer 3RH, by assuming that the c-axis length of the hexagonal crystal system graphite layer 2H is 2/3 times the c-axis length of the rhombohedral crystal system graphite layer 3RH in the graphite raw material. In this manner, the zero-point of the diffraction angle can be specified from the peak position. The zero-point of the diffraction angle is preferably specified using peak positions of diffracted beams via a plurality of c-axis direction lattice planes. For example, when using two peak positions, simultaneous equations may be solved. Further, when using three or more peak positions, regression analysis of a least-squares method or the like can be used. In this manner, the zero-point of the diffraction angle is able to be precisely specified by eliminating errors. In addition, it is also possible to specify the zero-point by using a single peak position, and in this case, it is possible to suppress the influence of errors to be small by using a high-order peak.

The integrated intensity calculation section 260 calculates the peak positions specific to the hexagonal crystal system graphite layer 2H and the rhombohedral crystal system graphite layer 3RH from the specified zero-point, by assuming that the a-axis and b-axis lengths of the hexagonal crystal system graphite layer 2H in graphite raw material are equal to the a-axis and b-axis lengths of the rhombohedral crystal system graphite layer 3RH therein, respectively; and the c-axis length of the hexagonal crystal system graphite layer 2H is 2/3 times the c-axis length of the rhombohedral crystal system graphite layer 3RH. In this manner, the peak separation is surely enabled by a powder X-ray diffraction pattern with respect to graphite raw material. As a result of this, ratios of the rhombohedral crystal system graphite layer 3RH and the hexagonal crystal system graphite layer 2H in the graphite raw material can be certainly measured, and thus it can be accurately and precisely determined whether the graphite raw material is a graphene precursor or not. By doing this, it becomes possible to produce graphene at low cost by using a graphene peeling method.

Then, the integrated intensity calculation section 260 calculates an integrated intensity at the calculated peak position, based on the result obtained by the fitting execution section 240. A function whose peak is represented, that corresponds to an X-ray diffraction peak based on a given initial value is subjected to fitting by the fitting execution section 240. For example, in the case where the peak position of the main peak is specified, a relatively wide angle range can be subjected to fitting by the entire pattern decomposition, based on information of a crystal system and a lattice constant. Further, in the case where the integrated intensity is calculated, fitting using a Lorentz function can be conducted.

The determination section 270 determines whether graphite raw material is a graphene precursor or not, based on the calculated integrated intensity. In the determination step, preferably determined is whether or not a rate of an integrated intensity value of a 101 diffracted beam of the rhombohedral crystal system graphite layer 3RH to an integrated intensity value of a 101 diffracted beam of the hexagonal crystal system graphite layer 2H at the calculated peak positions is same as or higher than a predetermined value. In this manner, graphite raw material for which a graphene peeling method is applicable can be distinguished using an easily comparable integrated intensity value.

Specifically, it is preferred to determine whether or not P3/(P3 + P4) is 0.31 or more when the calculated integrated intensity value of the 101 diffracted beam of the rhombohedral crystal system graphite layer 3RH represents P3, and the calculated integrated intensity value of the 101 diffracted beam of the hexagonal crystal system graphite layer 2H represents P4. In this manner, the graphite raw material for which a graphene peeling method is applicable can be clearly distinguished by using specific numerical values.

The output section 280 determinably outputs whether the graphite raw material is a graphene precursor or not, based on the calculated integrated intensity. For example, the integrated intensity value P3 of the 101 diffracted beam of the rhombohedral crystal system graphite layer 3RH and the integrated intensity value P4 of the 101 diffracted beam of the hexagonal crystal system graphite layer 2H, respectively, can be displayed on a display by the output section 280 to be able to be compared. Further, the output section 280 may display the above-described rate P3/(P3 + P4) of the integrated intensities. In this case, user himself/herself determines whether the sample is a graphene precursor or not with displaying as a determination material. On the other hand, the output section 280 may display the result from which whether the sample is a graphene precursor or not is distinguished by the determination section 270, and thus in that case, the user does not have a load of distinguishing.

[Method of distinguishing graphene precursor]

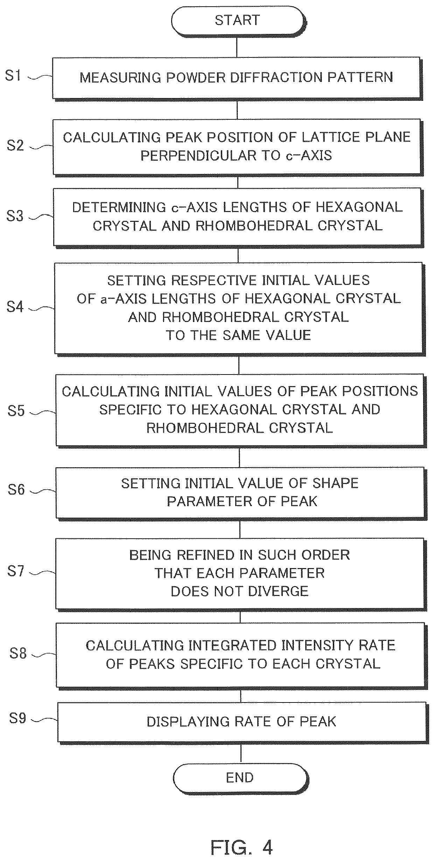

It can be determined whether graphite raw material is a graphene precursor or not by using the distinguishing system 10 constituted as described above. FIG. 4 is a flowchart showing a distinguishing method of a graphene precursor. The explanation is made from a stage of preparing powder X-ray diffraction data, so as to be easily understood.

First, graphite raw material is prepared. The graphite raw material used as a graphene precursor can be produced by a processing by radio-wave force and a processing by physical force in vacuum or air. That is, a graphite system carbon material more abundantly containing the rhombohedral crystal system graphite layer 3RH can be obtained by using the processing by the radio-wave force such as microwave, millimeter wave, plasma, electromagnetic induction heating (IH), magnetic field or the like, and the processing by the physical force such as ball mill, jet mill, centrifugal force, supercriti_cality or the like together in vacuum or air for a natural graphite material. It is preferred to apply the operations by the radio-wave force and the physical force are preferably applied thereto at the same time, however the radio-wave force and the physical force may be alternately applied thereto at each of predetermined intervals.

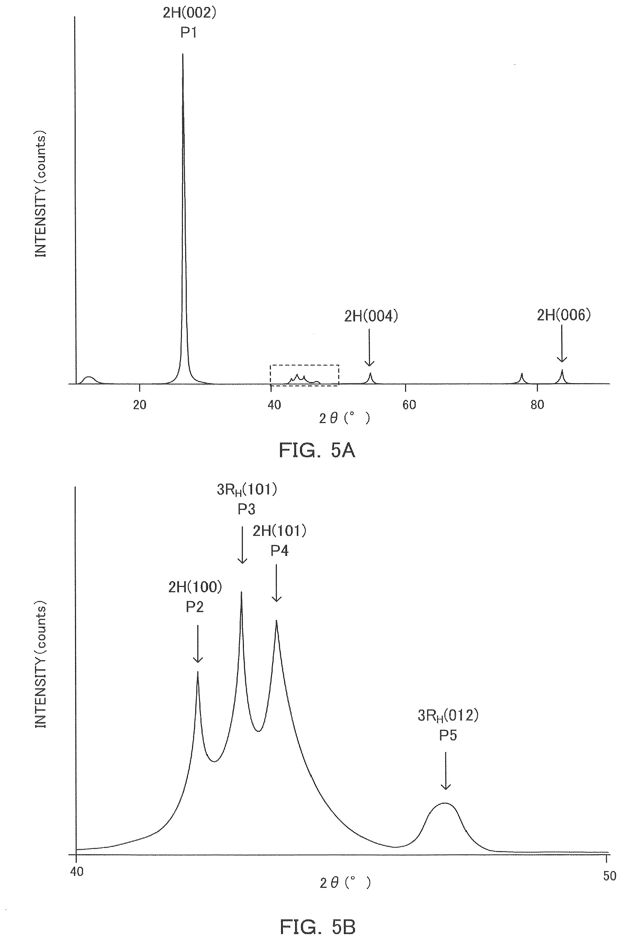

The powder X-ray diffraction pattern of graphite raw material prepared in this manner is measured (step S1). At that time, it is preferred to be measured up to a 2θ of 90° or more. FIGS. 5A and 5B are graphs schematically showing powder X-ray diffraction pattern profiles of graphite raw material with respect to a 2θ of 0 to 90° and a 2θ of 40 to 45°, respectively. FIG. 5B is a graph obtained by enlarging the inside of a dashed line in FIG. 5A. According to the actual measurement, peaks are hardly distinguishable as shown in FIGS. 5A and 5B, however schematic graphs are used to be easily understood.

Based on the resulting powder X-ray diffraction pattern, the peak positions of 002, 004 and 006 diffracted beams of graphite are calculated by the entire pattern decomposition (step S2). The entire pattern decomposition is a method of performing profile fitting in a wide angle range, based on information of a crystal system and a lattice constant.

In this manner, respective c-axis lengths of the hexagonal crystal system graphite layer 2H and the rhombohedral crystal system graphite layer 3RH in graphite are determined (step S3), and the zero-point of 2θ is specified. Then, respective initial values of the a-axis lengths of the hexagonal crystal system graphite layer 2H and the rhombohedral crystal system graphite layer 3RH in graphite are set to the same value (step S4). The initial values at four peak positions in a 2θ of 42 to 46° are calculated, based on the initial values of the hexagonal crystal system graphite layer 2H and the rhombohedral crystal system graphite layer 3RH in graphite (step S5). At this time, it is preferred that data in a 2θ of 40 to 50° is cut out from the viewpoint of processing efficiency. Further, respective initial values of shape parameters of four peaks are set (step S6).

Profile fitting is performed in optimal order so as not to prevent each parameter from diverging, and each parameter is refined (step S7). An integrated intensity rate Rate (3RH) = P3/(P3 + P4) of respective 101 peaks of the rhombohedral crystal system graphite layer 3RH and the hexagonal crystal system graphite layer 2H in graphite, obtained in this manner, is calculated (step S8). Then, a value of a rate of the acquired two peaks, Rate (3RH), is displayed (step S9). In addition, a peak representing the rhombohedral crystal system graphite layer 3RH, and a peak representing the hexagonal crystal system graphite layer 2H can be selected, however it is preferred to use P3 and P4. It is preferred that a value of Rate (3RH) = P3/(P3 + P4) is automatically displayed powder X-ray diffraction data of graphite raw material are specified.

In addition, according to the step S7, profile fitting for four peaks is performed in the following step. That is, scale factors are refined, and FWHMs (full widths at half maximum) of P2 and P3 are refined. At this time, it is preferred that respective FWHMs of P4 and P5 each are the same value as that of P3. Then, respective peak positions of P2 to P5 are refined. Further, a series of operations in steps S2 to S9 described above can be performed by executing a program.

[EXAMPLE]

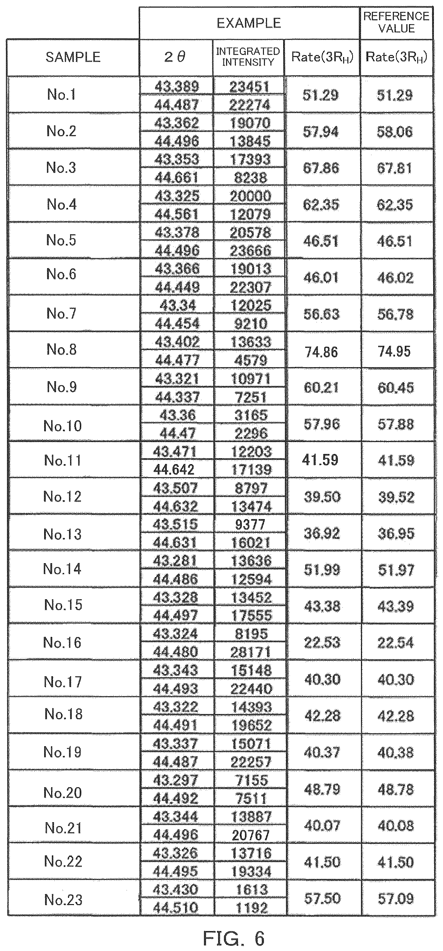

The sample data were measured using the above-described distinguishing method of a graphene precursor to find a value of Rate (3RH). FIG. 6 is a diagram showing a table that shows experimental results of each sample. Each sample used was provided by Graphene Platform Corporation, and the data of the provider are shown as reference values.

According to profile fitting when finding Rate (3RH), initial values at peak positions were determined from some literature values (theoretical values of bonding distance) and 100 or more actual measurement data as a = b = 2.46 angstroms and c = 6.71 angstroms for 2H. Then, the values each at which only the FWHM of P2 is narrow were utilized as initial values in comparison with other peaks. This is because it is determined that there are many cases where only P2 is viewed in actual measurement data, and thus the narrow FWHM is often observable in comparison with three other peaks. Eventually, the FWHM of P2 is independently refined, and thus it is enough to be narrower than that of each of P3 to P5 and initial values do not have to be set smaller than necessary.

In the case of distinguishing a graphene precursor, it appears that those having various crystallite sizes are mixed, from the viewpoint of properties of a graphite sample. In such a case, it appears that a precise fitting result is given by a Lorentz function. Thus, the Lorentz function was used as a profile function.

In this manner, the value of Rate (3RH) was able to be calculated accurately even from a powder X-ray diffraction pattern from which four peaks to be separated cannot be identified at all, from the measurement to the analysis in an automatic manner. As shown in FIG. 6, measurement values of Rate (3RH) close to approximately a reference value were obtained. In addition, the Lorentz function is used in the above-described Example, however profile fitting is enabled even by using an FP (Fundamental Parameter) method.

**EXPLANATION OF THE SYMBOLS**

| | |
|---|---|
| 10 | Distinguishing system |
| 100 | X-ray diffractometer |
| 110 | Goniometer |
| 120 | X-ray source arm |
| 121 | X-ray source |
| 122 | X-ray generator |
| 125 | Divergence slit |
| 130 | Detector arm |
| 131 | X-ray detector |
| 135 | Scattering slit |
| 137 | Receiving slit |
| 150 | Sample stage |

| 155 | Sample holder |
| 200 | Distinguishing apparatus |
| 200m | Memory |
| 200p | Processor |
| 210 | Data management section |
| 220 | Input section |
| 230 | Zero-point specification section |
| 240 | Fitting execution section |
| 260 | Integrated intensity calculation section |
| 270 | Determination section |
| 280 | Output section |
| S | Sample |

**Claims**

1. A distinguishing method of a graphene precursor with respect to graphite raw material in which a hexagonal crystal system graphite layer and a rhombohedral crystal system graphite layer are mixed, the method comprising the steps of:

specifying a zero-point of a diffraction angle from at least one peak position of a diffracted beam by lattice planes of c-axis direction for powder X-ray diffraction data of the graphite raw material;

calculating peak positions specific to the hexagonal crystal system graphite layer and the rhombohedral crystal system graphite layer from the specified zero-point, by assuming that an interplanar spacing between graphene sheets that form the graphite raw material is the same in any graphite layer mixed in the graphite raw material and that a crystal structure inside the graphene sheets is the same in any graphite layer mixed in the graphite raw material, wherein the a-axis and b-axis lengths of the hexagonal crystal system graphite layer 2H in the graphite raw material are equal to the a-axis and b-axis lengths of the rhombohedral crystal system graphite layer 3RH therein, respectively; and the c-axis length of the hexagonal crystal system graphite layer 2H is 2/3 times the c-axis length of the rhombohedral crystal system graphite layer 3RH;

calculating integrated intensities at the peak positions refined by profile fitting, with the calculated peak positions as initial values, based on the profile fitting; and

determining whether or not a rate of an integrated intensity value of a 101 diffracted beam of the hexagonal crystal system graphite layer to an integrated intensity value of a 101 diffracted beam of the rhombohedral crystal system graphite layer at the calculated peak positions is the same as or higher than a predetermined value, based on the calculated integrated intensities; and

determinably outputting that the graphite raw material is the graphene precursor when it is determined that the rate with regard to the integrated intensity values is the same as or higher than the predetermined value, and that the graphite raw material is not the graphene precursor when it is determined that the rate with regard to the integrated intensity values is lower than the predetermined value.

2. The distinguishing method of a graphene precursor according to claim 1, wherein in the step of specifying the zero-point of the diffraction angle, a peak position of a diffracted beam with the graphene sheets as lattice planes is used, by assuming that the interplanar spacing between a plurality of the graphene sheets that form the graphite raw material is the same in any structure mixed in the graphite raw material.

3. The distinguishing method of a graphene precursor according to claim 1 or 2, wherein it is determined whether or not P3/(P3 + P4) is 0.31 or more when the calculated integrated intensity value of the 101 diffracted beam of the rhombohedral crystal system graphite layer represents P3, and the calculated integrated intensity value of the 101 diffracted beam of the hexagonal crystal system graphite layer represents P4; and it is determined that the graphite raw material is the graphene precursor when the P3/(P3 + P4) is 0.31 or more, and it is determined that the graphite raw material is not the graphene precursor when the P3/(P3 + P4) is lower than 0.31.

4. A distinguishing apparatus (200) of a graphene precursor with respect to graphite raw material in which a hexagonal crystal system graphite layer and a rhombohedral crystal system graphite layer are mixed, the apparatus comprising:

a zero-point specification section (230) that specifies a zero-point of a diffraction angle from at least one peak position of a diffracted beam by lattice planes of c-axis direction for powder X-ray diffraction data of the graphite raw material;

a peak position calculation section that calculates peak positions specific to the hexagonal crystal system graphite layer and the rhombohedral crystal system graphite layer from the specified zero-point, by assuming that an interplanar spacing between graphene sheets that form the graphite raw material is the same in any graphite layer mixed in the graphite raw material and that a crystal structure inside the graphene sheets is the same in any graphite layer mixed in the graphite raw material, wherein the a-axis and b-axis lengths of the hexagonal crystal system graphite layer 2H in the graphite raw material are equal to the a-axis and b-axis lengths of the rhombohedral crystal system graphite layer 3RH therein, respectively; and the c-axis length of the hexagonal crystal system graphite layer 2H is 2/3 times the c-axis length of the rhombohedral crystal system graphite layer 3RH;

an integrated intensity calculation section (260) that calculates integrated intensities at the peak positions refined by profile fitting, with the calculated peak positions as initial values, based on the profile fitting;

a determination section (270) that determines whether or not a rate of an integrated intensity value of a 101 diffracted beam of the hexagonal crystal system graphite layer to an integrated intensity value of a 101 diffracted beam of the rhombohedral crystal system graphite layer at the calculated peak positions is the same as or higher than a predetermined value, based on the calculated integrated intensities;

an output section (280) that determinably outputs that the graphite raw material is the graphene precursor when it is determined that the rate with regard to the integrated intensity values is the same as or higher than the predetermined value, and that the graphite raw material is not the graphene precursor when it is determined that the rate with regard to the integrated intensity values is lower than the predetermined value.

5. A distinguishing program of a graphene precursor with respect to graphite raw material in which a hexagonal crystal system graphite layer and a rhombohedral crystal system graphite layer are mixed, the program causing a computer to execute the processes of:

specifying a zero-point of a diffraction angle from at least one peak position of a diffracted beam by lattice planes of c-axis direction for powder X-ray diffraction data of the graphite raw material;

calculating peak positions specific to the hexagonal crystal system graphite layer and the rhombohedral crystal system graphite layer from the specified zero-point, by assuming that an interplanar spacing between graphene sheets that form the graphite raw material is the same in any graphite layer mixed in the graphite raw material and that a crystal structure inside the graphene sheets is the same in any graphite layer mixed in the graphite raw material, wherein the a-axis and b-axis lengths of the hexagonal crystal system graphite layer 2H in the graphite raw material are equal to the a-axis and b-axis lengths of the rhombohedral crystal system graphite layer 3RH therein, respectively; and the c-axis length of the hexagonal crystal system graphite layer 2H is 2/3 times the c-axis length of the rhombohedral crystal system graphite layer 3RH;

calculating integrated intensities at the peak positions refined by profile fitting, with the calculated peak positions as initial values, based on the profile fitting;

determining whether or not a rate of an integrated intensity value of a 101 diffracted beam of the hexagonal crystal system graphite layer to an integrated intensity value of a 101 diffracted beam of the rhombohedral crystal system graphite layer at the calculated peak positions is the same as or higher than a predetermined value, based on the calculated integrated intensities; and

determinably outputting that the graphite raw material is the graphene precursor when it is determined that the rate with regard to the integrated intensity values is the same as or higher than the predetermined value, and that the graphite raw material is not the graphene precursor when it is determined that the rate with regard to the integrated intensity values is lower than the predetermined value.

## Patentansprüche

1. Unterscheidungsverfahren für einen Graphen-Vorläufer in Bezug auf ein Graphit-Rohmaterial, in dem eine Hexagonalkristallsystem-Graphit-Schicht und eine Rhombohedralkristallsystem-Graphit-Schicht gemischt sind, wobei das Verfahren die Schritte umfasst:

Spezifizieren eines Null-Punkts eines Beugungswinkels von wenigstens einer Peak-Position eines gebeugten Strahls durch Gitterebenen einer c-Achsen-Richtung für Pulver-Röntgen-Beugungsdaten des Graphit-Rohmaterials;

Berechnen von Peak-Positionen, die spezifisch für die Hexagonalkristallsystem-Graphit-Schicht und die Rhombohedralkristallsystem-Graphit-Schicht sind, aus dem spezifizierten Null-Punkt, durch Annehmen, dass ein Interplanarabstand zwischen Graphen-Lagen, die das Graphit-Rohmaterial bilden, in jeglicher in dem Graphit-Rohmaterial gemischter Graphit-Schicht gleich ist und dass eine Kristallstruktur innerhalb der Graphen-Lagen in jeglicher in dem Graphit-Rohmaterial gemischter Graphit-Schicht gleich ist, wobei die a-Achsen- und b-Achsen-Längen der Hexagonalkristallsystem-Graphit-Schicht 2H in dem Graphit-Rohmaterial gleich den a-Achsen- bzw. b-Achsen-Längen der Rhombohedralkristallsystem-Graphit-Schicht 3RH darin sind; und die c-Achsen-Länge der Hexagonalkristallsystem-Graphit-Schicht 2H 2/3-mal die c-Achsen-Länge der Rhombohedralkristallsystem-Graphit-Schicht 3RH ist;

Berechnen integrierter Intensitäten an den durch Profilfitten verfeinerten Peak-Positionen, mit den berechneten Peak-Positionen als anfängliche Werte, basierend auf dem Profilfitten; und

Bestimmen, ob oder nicht eine Rate eines integrierten Intensitätswerts eines 101-gebeugten Strahls der Hexagonalkristallsystem-Graphit-Schicht zu einem integrierten Intensitätswert eines 101-gebeugten Strahls der Rhombohedralkristallsystem-Graphit-Schicht an den berechneten Peak-Positionen gleich einem oder höher als ein vorgegebener Wert ist, basierend auf den berechneten integrierten Intensitäten; und

bestimmbar Ausgeben, dass das Graphit-Rohmaterial der Graphen-Vorläufer ist, wenn bestimmt wird, dass die Rate hinsichtlich der integrierten Intensitätswerte gleich dem oder höher als der vorgegebene Wert ist, und dass das Graphit-Rohmaterial nicht der Graphen-Vorläufer ist, wenn bestimmt wird, dass die Rate hinsichtlich der integrierten Intensitätswerte niedriger als der vorgegebene Wert ist.

2. Unterscheidungsverfahren für einen Graphen-Vorläufer nach Anspruch 1, wobei beim Schritt des Spezifizierens des Null-Punkts des Beugungswinkels eine Peak-Position eines gebeugten Strahls mit den Graphen-Lagen als Gitterebenen verwendet wird, durch Annehmen, dass der Interplanarabstand zwischen einer Vielzahl der Graphen-Lagen, die das Graphit-Rohmaterial bilden, in jeglicher in dem Graphit-Rohmaterial gemischten Struktur gleich ist.

3. Unterscheidungsverfahren für einen Graphen-Vorläufer nach Anspruch 1 oder 2,
wobei bestimmt wird, ob oder nicht P3/(P3 + P4) 0,31 oder mehr ist, wenn der berechnete integrierte Intensitätswert des 101-gebeugten Strahls der Rhombohedralkristallsystem-Graphit-Schicht P3 repräsentiert, und der berechnete integrierte Intensitätswert des 101-gebeugten Strahls der Hexagonalkristallsystem-Graphit-Schicht P4 repräsentiert; und bestimmt wird, dass das Graphit-Rohmaterial der Graphen-Vorläufer ist, wenn P3/(P3 + P4) 0,31 oder mehr ist, und bestimmt wird, dass das Graphit-Rohmaterial nicht der Graphen-Vorläufer ist, wenn P3/(P3 + P4) niedriger als 0,31 ist.

4. Unterscheidungsvorrichtung (200) für einen Graphen-Vorläufer in Bezug auf ein Graphit-Rohmaterial, in dem eine Hexagonalkristallsystem-Graphit-Schicht und eine Rhombohedralkristallsystem-Graphit-Schicht gemischt sind, wobei die Vorrichtung umfasst:

eine Null-Punkt-Spezifizierungs-Sektion (230), die einen Null-Punkt eines Beugungswinkels von wenigstens einer Peak-Position eines gebeugten Strahls durch Gitterebenen einer c-Achsen-Richtung für Pulver-Röntgen-Beugungsdaten des Graphit-Rohmaterials spezifiziert;

eine Peak-Position-Berechnungs-Sektion, die Peak-Positionen, die spezifisch für die Hexagonalkristallsystem-Graphit-Schicht und die Rhombohedralkristallsystem-Graphit-Schicht sind, von dem spezifizierten Null-Punkt berechnet, durch Annehmen, dass ein Interplanarabstand zwischen Graphen-Lagen, die das Graphit-Rohmaterial bilden, in jeglicher in dem Graphit-Rohmaterial gemischter Graphit-Schicht gleich ist und dass eine Kristallstruktur innerhalb der Graphen-Lagen in jeglicher in dem Graphit-Rohmaterial gemischter Graphit-Schicht gleich ist, wobei die a-Achsen- und b-Achsen-Längen der Hexagonalkristallsystem-Graphit-Schicht 2H in dem Graphit-Rohmaterial gleich den a-Achsen- bzw. b-Achsen-Längen der Rhombohedralkristallsystem-Graphit-Schicht 3RH darin sind; und die c-Achsen-Länge der Hexagonalkristallsystem-Graphit-Schicht 2H 2/3-mal die c-Achsen-Länge der Rhombohedralkristallsystem-Graphit-Schicht 3RH ist;

eine Berechnungssektion für eine integrierte Intensität (260), die integrierte Intensitäten an den durch Profilfitten verfeinerten Peak-Positionen, mit den berechneten Peak-Positionen als anfängliche Werte, basierend auf dem Profilfitten, berechnet;

eine Bestimmungssektion (270), die bestimmt, ob oder nicht eine Rate eines integrierten Intensitätswerts eines 101-gebeugten Strahls der Hexagonalkristallsystem-Graphit-Schicht zu einem integrierten Intensitätswert eines 101-gebeugten Strahls der Rhombohedralkristallsystem-Graphit-Schicht an den berechneten Peak-Positionen gleich einem oder höher als ein vorgegebener Wert ist, basierend auf den berechneten integrierten Intensitäten;

eine Ausgabesektion (280), die bestimmbar ausgibt, dass das Graphit-Rohmaterial der Graphen-Vorläufer ist, wenn bestimmt wird, dass die Rate hinsichtlich der integrierten Intensitätswerte gleich dem oder höher als der vorgegebene Wert ist, und dass das Graphit-Rohmaterial nicht der Graphen-Vorläufer ist, wenn bestimmt wird, dass die Rate hinsichtlich der integrierten Intensitätswerte niedriger als der vorgegebene Wert ist.

5. Unterscheidungsprogramm für einen Graphen-Vorläufer in Bezug auf ein Graphit-Rohmaterial, in dem eine Hexagonalkristallsystem-Graphit-Schicht und eine Rhombohedralkristallsystem-Graphit-Schicht gemischt sind, wobei das Programm bewirkt, dass ein Computer die Prozesse ausführt:

eines Spezifizierens eines Null-Punkts eines Beugungswinkels von wenigstens einer Peak-Position eines gebeugten Strahls durch Gitterebenen einer c-Achsen-Richtung für Pulver-Röntgen-Beugungsdaten des Graphit-Rohmaterials;

eines Berechnens von Peak-Positionen, die spezifisch für die Hexagonalkristallsystem-Graphit-Schicht und die Rhombohedralkristallsystem-Graphit-Schicht sind, aus dem spezifizierten Null-Punkt, durch Annehmen, dass ein Interplanarabstand zwischen Graphen-Lagen, die das Graphit-Rohmaterial bilden, in jeglicher in dem Graphit-Rohmaterial gemischter Graphit-Schicht gleich ist und dass eine Kristallstruktur innerhalb der Graphen-Lagen in jeglicher in dem Graphit-Rohmaterial gemischter Graphit-Schicht gleich ist, wobei die a-Achsen- und b-Achsen-Längen der Hexagonalkristallsystem-Graphit-Schicht 2H in dem Graphit-Rohmaterial gleich den a-Achsen- bzw. b-Achsen-Längen der Rhombohedralkristallsystem-Graphit-Schicht 3RH darin sind; und die c-Achsen-Länge der Hexagonalkristallsystem-Graphit-Schicht 2H 2/3-mal die c-Achsen-Länge der Rhombohedralkristallsystem-Graphit-Schicht 3RH ist;

eines Berechnens integrierter Intensitäten an den durch Profilfitten verfeinerten Peak-Positionen, mit den berechneten Peak-Positionen als anfängliche Werte, basierend auf dem Profilfitten;

eines Bestimmens, ob oder nicht eine Rate eines integrierten Intensitätswerts eines 101-gebeugten Strahls der Hexagonalkristallsystem-Graphit-Schicht zu einem integrierten Intensitätswert eines 101-gebeugten Strahls der Rhombohedralkristallsystem-Graphit-Schicht an den berechneten Peak-Positionen gleich einem oder höher als ein vorgegebener Wert ist, basierend auf den berechneten integrierten Intensitäten; und

eines bestimmbaren Ausgebens, dass das Graphit-Rohmaterial der Graphen-Vorläufer ist, wenn bestimmt wird, dass die Rate hinsichtlich der integrierten Intensitätswerte gleich dem oder höher als der vorgegebene Wert ist, und dass das Graphit-Rohmaterial nicht der Graphen-Vorläufer ist, wenn bestimmt wird, dass die Rate hinsichtlich der integrierten Intensitätswerte niedriger als der vorgegebene Wert ist.

## Revendications

1. Procédé de distinction d'un précurseur de graphène par rapport à une matière première graphite dans laquelle une couche de graphite à système cristallin hexagonal et une couche de graphite à système cristallin rhomboédrique sont mélangées, le procédé comprenant les étapes :

spécifier un point zéro d'un angle de diffraction à partir d'au moins une position de pic d'un faisceau diffracté par des plans réticulaires de sens de l'axe c pour des données de diffraction sur poudre de rayons X de la matière première graphite ;

calculer des positions de pics spécifiques à la couche de graphite à système cristallin hexagonal et à la couche de graphite à système cristallin rhomboédrique à partir du point zéro spécifié, en faisant l'hypothèse qu'une distance inter-réticulaire entre des feuilles de graphène qui forment la matière première graphite est la même dans n'importe quelle couche de graphite mélangée dans la matière première graphite et qu'une structure cristalline à l'intérieur des feuilles de graphène est la même dans n'importe quelle couche de graphite mélangée dans la matière première graphite, dans laquelle les longueurs selon l'axe a et l'axe b de la couche de graphite à système cristallin hexagonal 2H dans la matière première graphite sont respectivement égales aux longueurs selon l'axe a et l'axe b de la couche de graphite à système cristallin rhomboédrique 3RH à l'intérieur ; et la longueur selon l'axe c de la couche de graphite à système cristallin hexagonal 2H représente les 2/3 de la longueur selon l'axe c de la couche de graphite à système cristallin rhomboédrique 3RH ;

calculer les intensités intégrées au niveau des positions de pics affinées par ajustement de profil, avec les positions de pics calculées comme valeurs initiales, sur la base de l'ajustement de profil ; et

déterminer si ou pas un taux d'une valeur d'intensité intégrée d'un faisceau diffracté (101) de la couche de graphite à système cristallin hexagonal à une valeur d'intensité intégrée d'un faisceau diffracté (101) de la couche de graphite à système cristallin rhomboédrique aux positions de pics calculées est supérieur ou égal à une valeur prédéterminée, sur la base des intensités intégrées calculées ; et

conclure de manière déterminante que la matière première graphite est le précurseur de graphène lorsqu'il est déterminé que le taux concernant les valeurs d'intensité intégrée est supérieur ou égal à la valeur prédéterminée, et que la matière première graphite n'est pas le précurseur de graphène lorsqu'il est déterminé que le taux concernant les valeurs d'intensité intégrée est inférieur à la valeur prédéterminée.

2. Procédé de distinction d'un précurseur de graphène selon la revendication 1,
dans lequel dans l'étape de spécification du point zéro de l'angle de diffraction, une position de pic d'un faisceau diffracté avec des feuilles de graphène comme plans réticulaires est utilisée, en faisant l'hypothèse que la distance inter-réticulaire entre une pluralité des feuilles de graphène qui forment la matière première graphite est la même dans n'importe quelle structure mélangée dans la matière première graphite.

3. Procédé de distinction d'un précurseur de graphène selon la revendication 1 ou 2,
dans lequel il est déterminé si ou pas, P3/(P3 + P4) est 0,31 ou plus lorsque la valeur d'intensité intégrée calculée du faisceau diffracté (101) de la couche de graphite à système cristallin rhomboédrique représente P3, et que la valeur d'intensité intégrée calculée du faisceau diffracté (101) de la couche de graphite à système cristallin hexagonal représente P4 ; et il est déterminé que la matière première graphite est le précurseur de graphène lorsque P3/(P3 + P4) est de 0,31 ou plus, et il est déterminé que la matière première graphite n'est pas le précurseur de graphène lorsque P3/(P3 + P4) est inférieur à 0,31.

4. Appareil de distinction (200) d'un précurseur de graphène par rapport à une matière première graphite dans laquelle une couche de graphite à système cristallin hexagonal et une couche de graphite à système cristallin rhomboédrique sont mélangées, l'appareil comprenant :

   une section de spécification de point zéro (230) qui spécifie un point zéro d'un angle de diffraction à partir d'au moins une position de pic d'un faisceau diffracté par des plans réticulaires de sens de l'axe c pour des données de diffraction sur poudre de rayons X de la matière première graphite ;
   une section de calcul de position de pic qui calcule des positions de pics spécifiques à la couche de graphite à système cristallin hexagonal et à la couche de graphite à système cristallin rhomboédrique à partir du point zéro spécifié, en faisant l'hypothèse qu'une distance inter-réticulaire entre des feuilles de graphène qui forment la matière première graphite est la même dans n'importe quelle couche de graphite mélangée dans la matière première graphite et qu'une structure cristalline à l'intérieur des feuilles de graphène est la même dans n'importe quelle couche de graphite mélangée dans la matière première graphite, dans laquelle les longueurs selon l'axe a et l'axe b de la couche de graphite à système cristallin hexagonal 2H dans la matière première graphite sont respectivement égales aux longueurs selon l'axe a et l'axe b de la couche de graphite à système cristallin rhomboédrique 3RH à l'intérieur ; et la longueur selon l'axe c de la couche de graphite à système cristallin hexagonal 2H représente les 2/3 de la longueur selon l'axe c de la couche de graphite à système cristallin rhomboédrique 3RH ;
   une section de calcul d'intensité intégrée (260) qui calcule les intensités intégrées aux positions de pics affinées par ajustement de profil, avec les positions de pics calculées comme valeurs initiales, sur la base de l'ajustement de profil ;
   une section de détermination (270) qui détermine si ou pas un taux d'une valeur d'intensité intégrée d'un faisceau diffracté (101) de la couche de graphite à système cristallin hexagonal à une valeur d'intensité intégrée d'un faisceau diffracté (101) de la couche de graphite à système cristallin rhomboédrique au niveau des positions de pics calculées est supérieure ou égale à une valeur prédéterminée, sur la base des intensités intégrées calculées ;
   une section de sortie (280) qui conclut de manière déterminante que la matière première graphite est le précurseur de graphène lorsqu'il est déterminé que le taux concernant les valeurs d'intensité intégrée est supérieur ou égal à la valeur prédéterminée, et que la matière première graphite n'est pas le précurseur de graphène lorsqu'il est déterminé que le taux concernant les valeurs d'intensité intégrée est inférieur à la valeur prédéterminée.

5. Programme de distinction d'un précurseur de graphène par rapport à une matière première graphite dans laquelle une couche de graphite à système cristallin hexagonal et une couche de graphite à système cristallin rhomboédrique sont mélangées, le programme amenant un ordinateur à exécuter les procédés de :

   spécification d'un point zéro d'un angle de diffraction à partir d'au moins une position de pic d'un faisceau diffracté par des plans réticulaires de sens de l'axe c pour des données de diffraction sur poudre de rayons X de la matière première graphite ;
   calcul des positions de pics spécifiques à la couche de graphite à système cristallin hexagonal et à la couche de graphite à système cristallin rhomboédrique à partir du point zéro spécifié, en faisant l'hypothèse qu'une distance inter-réticulaire entre des feuilles de graphène qui forment la matière première graphite est la même dans n'importe quelle couche de graphite mélangée dans la matière première graphite et qu'une structure cristalline à l'intérieur des feuilles de graphène est la même dans n'importe quelle couche de graphite mélangée dans la matière première graphite, dans laquelle les longueurs selon l'axe a et l'axe b de la couche de graphite à système cristallin hexagonal 2H dans la matière première graphite sont respectivement égales aux longueurs selon l'axe a et l'axe b de la couche de graphite à système cristallin rhomboédrique 3RH à l'intérieur ; et la longueur selon l'axe c de la couche de graphite à système cristallin hexagonal 2H représente les 2/3 de la longueur selon l'axe c de la couche de graphite à système cristallin rhomboédrique 3RH ;

calcul des intensités intégrées au niveau des positions de pics affinées par ajustement de profil, avec les positions de pics calculées comme valeurs initiales, sur la base de l'ajustement de profil ;

détermination de si ou pas un taux d'une valeur d'intensité intégrée d'un faisceau diffracté (101) de la couche de graphite à système cristallin hexagonal à une valeur d'intensité intégrée d'un faisceau diffracté (101) de la couche de graphite à système cristallin rhomboédrique aux positions de pics calculées est supérieur ou égal à une valeur prédéterminée, sur la base des intensités intégrées calculées ; et

conclusion de manière déterminante que la matière première graphite est le précurseur de graphène lorsqu'il est déterminé que le taux concernant les valeurs d'intensité intégrée est supérieur ou égal à la valeur prédéterminée, et que la matière première graphite n'est pas le précurseur de graphène lorsqu'il est déterminé que le taux concernant les valeurs d'intensité intégrée est inférieur à la valeur prédéterminée.

FIG. 1A

FIG. 1B

FIG. 2

FIG. 3

START

S1 — MEASURING POWDER DIFFRACTION PATTERN

S2 — CALCULATING PEAK POSITION OF LATTICE PLANE
PERPENDICULAR TO c-AXIS

S3 — DETERMINING c-AXIS LENGTHS OF HEXAGONAL
CRYSTAL AND RHOMBOHEDRAL CRYSTAL

S4 — SETTING RESPECTIVE INITIAL VALUES
OF a-AXIS LENGTHS OF HEXAGONAL CRYSTAL
AND RHOMBOHEDRAL CRYSTAL
TO THE SAME VALUE

S5 — CALCULATING INITIAL VALUES OF PEAK POSITIONS
SPECIFIC TO HEXAGONAL CRYSTAL AND
RHOMBOHEDRAL CRYSTAL

S6 — SETTING INITIAL VALUE OF SHAPE
PARAMETER OF PEAK

S7 — BEING REFINED IN SUCH ORDER
THAT EACH PARAMETER
DOES NOT DIVERGE

S8 — CALCULATING INTEGRATED INTENSITY RATE
OF PEAKS SPECIFIC TO EACH CRYSTAL

S9 — DISPLAYING RATE OF PEAK

END

FIG. 4

FIG. 5A

FIG. 5B

| SAMPLE | EXAMPLE | | | REFERENCE VALUE |
|---|---|---|---|---|
| | $2\theta$ | INTEGRATED INTENSITY | Rate(3R$_H$) | Rate(3R$_H$) |
| No.1 | 43.389 | 23451 | 51.29 | 51.29 |
| | 44.487 | 22274 | | |
| No.2 | 43.362 | 19070 | 57.94 | 58.06 |
| | 44.496 | 13845 | | |
| No.3 | 43.353 | 17393 | 67.86 | 67.81 |
| | 44.661 | 8238 | | |
| No.4 | 43.325 | 20000 | 62.35 | 62.35 |
| | 44.561 | 12079 | | |
| No.5 | 43.378 | 20578 | 46.51 | 46.51 |
| | 44.496 | 23666 | | |
| No.6 | 43.366 | 19013 | 46.01 | 46.02 |
| | 44.449 | 22307 | | |
| No.7 | 43.34 | 12025 | 56.63 | 56.78 |
| | 44.454 | 9210 | | |
| No.8 | 43.402 | 13633 | 74.86 | 74.95 |
| | 44.477 | 4579 | | |
| No.9 | 43.321 | 10971 | 60.21 | 60.45 |
| | 44.337 | 7251 | | |
| No.10 | 43.36 | 3165 | 57.96 | 57.88 |
| | 44.47 | 2296 | | |
| No.11 | 43.471 | 12203 | 41.59 | 41.59 |
| | 44.642 | 17139 | | |
| No.12 | 43.507 | 8797 | 39.50 | 39.52 |
| | 44.632 | 13474 | | |
| No.13 | 43.515 | 9377 | 36.92 | 36.95 |
| | 44.631 | 16021 | | |
| No.14 | 43.281 | 13636 | 51.99 | 51.97 |
| | 44.486 | 12594 | | |
| No.15 | 43.328 | 13452 | 43.38 | 43.39 |
| | 44.497 | 17555 | | |
| No.16 | 43.324 | 8195 | 22.53 | 22.54 |
| | 44.480 | 28171 | | |
| No.17 | 43.343 | 15148 | 40.30 | 40.30 |
| | 44.493 | 22440 | | |
| No.18 | 43.322 | 14393 | 42.28 | 42.28 |
| | 44.491 | 19652 | | |
| No.19 | 43.337 | 15071 | 40.37 | 40.38 |
| | 44.487 | 22257 | | |
| No.20 | 43.297 | 7155 | 48.79 | 48.78 |
| | 44.492 | 7511 | | |
| No.21 | 43.344 | 13887 | 40.07 | 40.08 |
| | 44.496 | 20767 | | |
| No.22 | 43.326 | 13716 | 41.50 | 41.50 |
| | 44.495 | 19334 | | |
| No.23 | 43.430 | 1613 | 57.50 | 57.09 |
| | 44.510 | 1192 | | |

FIG. 6

FIG. 7A

FIG. 7B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016038692 W

- JP 5697067 B

**Non-patent literature cited in the description**

- **R. A. YOUNG.** The Rietveld Method'', International Union of Crystallography. Oxford University Press, 1995, 255-275